# EUROPEAN PATENT APPLICATION

(11) **EP 1 275 308 A1**
(43) Date of publication of application: **15.01.2003**
(21) Application number: 01117090.9
(22) Date of filing: 13.07.2001
(51) Int. Cl.: A23L 1/30, A23L 1/305, A61K 35/78, A61K 38/00, A61K 31/195

(54) **Food composition offering stress relaxation to mammals**

(71) Applicant: The Procter & Gamble Company, Cincinnati, Ohio 45202 (US)
(72) Inventor: Fischer, Christa Maria, 65760 Eschborn (DE); Weber, Regina Brigitte, 61449 Steinbach (DE)
(74) Representative: Kremer, Véronique

(57) **Abstract**

The present invention provides a food composition, preferably for use as beverage or other liquid food, which delivers a stress-alleviating effect to mammals, especially humans. The active ingredients of this composition are protein fractions and herbal extracts.

## Description

### Field of the invention

The subject of the present invention is a food composition, preferably an aqueous beverage, comprising a mixture of protein fractions and herbal extracts. This mixture causes, when taken up via the digestive system, stress relaxation to mammals, especially humans.

### Background of the invention

A major part of the healthy population is reporting sleep dissatisfaction more or less frequently. The reasons therefore are e.g. internal restlessness or stress. The latter reason is gaining more and more importance especially in the working population as the professional workloads for individuals continue to increase significantly through the last decades. Because of poor and insufficient sleep the daily demands are perceived as an even greater burden. Thereby, additional stress is generated and a vicious circle starts.

Several substances for providing a certain degree of relaxation are known. One prominent example is the amino acid tryptophan (Trp). The journal of nutrition, 1992, 122(9), 1781-1791, discloses that L-Trp intake influences the sleep latency of infants. Prevention, 1988, 40(1), 114, 116-117, discloses that Trp may influence sleep, appetite or pain perception and suggests the use of Trp for e.g. the correction of insomnia. Nutrition reviews, 1986, 44(suppl.), 70-73, discloses, based on animal studies, that L-Trp, but not other amino acids, increases sleepiness and induces sleep. Nutrition and health, 3(1/2), 55-67, 33 ref., investigates the effects of Trp on serotonin synthesis, on sleep and on pain. In WO-A-99/55174 a protein supplement, having a ratio of L-Trp to neutral amino acids of at least 0.06, is disclosed for readily crossing the blood-brain barrier and treating serotoninergic system dysfunctions, such as stress-related depression and sleep disorders. EP-A-951842 teaches a product for the complete nutrition of an infant, providing 1.7-3.5 g Trp per 100 g protein and having a weight ratio of Trp to the sum of the large neutral amino acids of 4.8-10:100. The disclosed product is suggested for improving senses of well-being or sleeping behaviour. US 4897380 discloses a mixture of L-Trp and fructose as a dietary supplement for daily use for promoting sleep and relieving chronic pain. JP-A-61152623 discloses the use of L-Trp to get rid from stress. US 4419345 a composition, comprising a Trp salt and calcium cations, for inducting sleep. A Trp-free composition for increasing the amount of stage 4 sleep is disclosed in EP-A-144051 as a dietetic supplement.

Protein fragments, especially peptides from milk, are also known in the art as for stress-relaxation purposes. RU-C-2161500 discloses a milk protein fragment, namely with the amino acid sequence Tyr-Pro-Phe-Pro-Gly-Pro-lle, for anxiolytic use. EP-B-714910 discloses peptides from casein hydrolysate, especially Tyr-Leu-Gly-Tyr-Leu-Glu-Gln-Leu-Leu-Arg, for use as sedatives. JP-A-2182169 teaches a peptide mixture from hydrolysed cow milk casein for use as food to relax excess tension. In JP-A-61277697 peptides from human milk, hydrolysed by pepsin, are suggested as sedatives.

Another component of compositions for stress relieve is theanine (L-γ-glutamylethylamide), an amino acid, which is accessible from tealeaves, which contain theanine in an amount of 1-2%. WO-A-94/42096 mentions theanine to relieve insomnia. JP-A-9012454 teaches theanine as an active compound for intensification of appearance and preservation of α wave, which is related to mental relaxation. In WO-A-99/42096 theanine is disclosed to aid relieve of e.g. insomnia.

Also combinations of peptides or amino acids with herbal extracts, such as hops extract or valerian extract, are known. JP-A-60202825 discloses herb extracts, e.g. valerian, together with Trp as narcotic and sedative actives. In WO-A-00/59519 the use of colostral milk products/extracts together with plant components/extracts and minerals/-salts for e.g. relaxing is taught. WO-A-99/61038 discloses a mixture of L-Trp with e.g. hops, green tea extract or milk for alleviating stress.

Current, commercially available means for providing relaxation for better sleep can be structured by two main groups: tablets and herbal teas. Tablets are not perceived as natural by the consumer and are always regarded as something non-natural or chemical. The problem of herbal teas is that their preparation is not convenient enough, e.g. prior to going to bed and also the taste is in many cases not perceived as satisfactory. Another problem is that by drinking such a tea a larger quantity of liquid is taken up by the body, which can cause involuntary waking up at night for urge to pass water. Thus, there is a lack of products for alleviating stress, which are perceived as natural, health-positive, pleasurable and convenient.

Therefore it would be favourable to provide a composition for relaxation of stress, which is substantially based on natural substances, which is convenient for use and which pleases the user when applying the composition. In particular with respect to convenience of use a preferred execution of such a composition could be a food composition, especially a beverage. Beverages have the advantage over solid food that they do not have to be dissolved and/or emulsified while passing the digestive system, prior to being taken up by the body. Thereby, no special body activity is required for processing a food, which is meant to act as a sedative.

Peptides from natural occurring proteins as well as herbal extracts, such as teas, fulfil the requirements of being natural and health-positive. It is a known problem that both peptides and herbal extracts have a strong taste, which at higher concentrations is perceived as annoying and unacceptable for e.g. humans. Trp and proteins have a bitter taste, which is known from agricultural and biological chemistry (Japan), 1988, 52(3), 819-827 and Lebensmittel - Wissenschaft und Technologie, 5(2), 47-50. The unpleasant taste prevents food compositions, such as beverages, from having incorporated higher concentrations of those active compounds. This means, just like in case of the aforementioned teas, that the consumer of such a beverage had to drink larger quantities for taking up amounts of actives sufficient for providing the intended relaxation effect. This can then lead to involuntary waking up at night for urge to pass water.

If the beverage, comprising the stress relaxation composition, is used for better sleep, it would be preferable to have a high concentration of active substances in the beverage in order to keep the volume of liquid low. By this, unwanted waking up at night can be prevented. But having high concentrations of an active compound in a conventional beverage results in an unacceptable taste.

Therefore, the problem the present invention seeks to solve is to provide a composition for mammals, especially humans, for relaxation of stress, which is perceived as natural, which is convenient for use and which is palatable to the consumer.

An additional problem the present invention in a preferred embodiment seeks to solve is to provide such a composition as a beverage or other liquid food, which has a sufficiently high stress-relaxing effect to relieve stress in a low consumption volume, e.g. not more than 150 g of liquid.

The aforementioned problems have been solved by combining peptides, which are known for relieving stresses, with herbal extracts, having a similar effect. Surprisingly it has been found, that an aqueous mixture of both classes of substances delivers a synergistic effect in terms of stress relieve. This means that the combination of e.g. 0.5 mg of peptide with 0.5 mg of a herbal extract in e.g. 10 ml of water has more than double the relaxing effect than single doses of 0.5 mg of peptide in 10 ml of water or 0.5 mg of a herbal extract in 10 ml of water would suggest. That means, the two classes of ingredients do not inhibit each other as those skilled in the art may expect, but amplify each other. Without wishing to be bound by theory it is speculated that the reason for the synergy is that peptides and herbal extracts are causing their relaxing effects via different mechanisms, e.g. by activating different receptors.

### Summary of the invention

The present invention provides a food composition, preferably for use as beverage or other liquid food, which delivers a stress-alleviating effect to mammals, especially humans. The active ingredients of this composition are protein fractions and herbal extracts.

### Detailed description of the invention

### a) Protein fractions

As used herein, a quantity in kg, g or mg is given as the weight of a substance in dry condition, e.g. as powder. If already solubilised, the dry weight has to be calculated.

The term "protein fractions" as used herein refers to peptides, which are obtained from proteins via hydrolysis. The hydrolysis can be facilitated by enzymes, such as pepsin, or by acids, such as mineral acids or organic acids. Particularly useful in the context of the present invention are oligopeptides, containing not more than 100 amino acids. Generally it can be assumed that by decreasing length of a peptide the water-solubility of this peptide increases, which is favourable for the uptake via the digestive system, especially in the context of beverages.

Exemplary protein fractions, which have proven particularly useful for the purpose of the present invention, are the amino acid theanine (e.g. "Suntheanine", Taiyo Kagaku Ltd. Yokkaichi City Mie, Japan), PRODIET F 240 (obtainable from Ingredia, Arras, France; details to be found in EP 714910), containing the relaxing peptide with the sequence Tyr-Leu-Gly-Tyr-Leu-Glu-Gln-Leu-Leu-Arg, and a delta sleep-inducing peptide (rabbit) (hereinafter DSIP) with the sequence Trp-Ala-Gly-Gly-Asp-Ala-Ser-Gly-Glu (e.g. from Bachem Peninsula Laboratories Inc., San Carlos, CA, USA; details in Merck index, page 544, compound 3445). All three mentioned peptides are known for sedative, stress-alleviating action. Further examples for suitable protein fractions with relaxing effects for use in the context of the present invention are casomorphins, caxoxines and lactrophins, which are all milk-derived peptides; more essentially, they have been derived from caseine and lactalbumin.

### b) Herbal extracts

As used herein, a quantity in kg, g or mg is given as the weight of a substance in dry condition, e.g. as powder. If already solubilised, the dry weight has to be calculated.

The term "herbal extracts" as used herein refers to essences of plants, which have been obtained via liquid extraction from at least parts of the respective plant. In most cases the extracting agent is water or mixtures of ethanol and water.

Exemplary herbal extracts for the purpose of the present invention are extracts of hops (Muster 1984/01, Lab.-Ch. 0910617 from Plantextrakt GmbH & Co. KG, Vestenbergsgreuth, Germany), balm mint (Art. 253029/1200286 from Plantextrakt GmbH & Co. KG, Vestenbergsgreuth, Germany) and camomile (Art. 253293/9202597 from Plantextrakt GmbH & Co. KG, Vestenbergsgreuth, Germany). Other herbal extracts useful in the context of the present invention are essences from valerian, lavender, schizandra, green oats, kava-kava, St. John's wort, griffonia, skullcap, ginger, orange blossom or passionflower. Those essences are broadly used and available from numerous suppliers.

### c) The composition

It is useful in the context of the present invention to define the concentration or the dosage of the active stress-relaxing composition in a foodstuff in the form of "servings". A serving is the amount of the foodstuff, which is usually eaten/drunk by a consumer for reliably achieving the result of stress relaxation. As the preferred execution of the composition of the present invention is a beverage, the serving can be defined as the overall amount of the liquid, which forms the beverage. Typically, a serving in accordance with the beverage of the present invention is between 80 and 150 g, preferably 125 g.

A serving of the present invention contains protein fractions in a total amount of 50 mg to 400 mg in case of theanine, 0.5 mg to 5 mg in case of DSIP and 50 mg to 750 mg in case of Prodiet F 240, containing 2 - 4% of Tyr-Leu-Gly-Tyr-Leu-Glu-Gln-Leu-Leu-Arg; and herbal extracts in a total amount of 10 mg to 1000 mg. Those amounts, contained in 125 g aqueous liquid, guarantee a stress-relaxing effect while still offering an acceptable and pleasurable taste of the beverage.

The ratio of protein fractions to herbal extracts ranges from 40 : 1 to 1 : 10000 and preferably from 40 : 1 to 1 :5000.

The composition of the present invention optionally can contain additional compounds. In the case of a beverage for instance milk, sugars (or sugar substitutes), other carbohydrates such as oligosaccharides, fruit juices, fruit- and/or vegetable extracts, flavours, colorants (such as β-carotin), vitamins, sources for minerals, e.g. calcium (such as calcium citrate malate), carbon dioxide or antioxidants and other stabilizers.

### d) Examples

An exemplary beverage according to the composition of the present invention is given in table 1.

**Table 1**

| **Ingredients** | **Weight %** |
|---|---|
| Inverted sugar | 6.534 |
| Acidified cloudifier | 5.0 |
| Pectin | 0.35 |
| Prodiet F240 | 0.1 |
| Balm mint extract | 0.45 |
| Hops extract | 0.2 |
| Ascorbic acid | 0.03 |
| Aspartame | 0.0055 |
| Acesulfam potassium | 0.011 |
| Flavourings / extracts | 0.183 |
| Water | 87.1365 |

## Claims

1. Composition for delivering relaxation to a mammal, especially a human, when consumed orally and taken up via the digestive system, said composition comprising two groups of functional ingredients:
(a) protein fractions selected from theanine, or peptides, preferably Tyr-Leu-Gly-Tyr-Leu-Glu-Gln-Leu-Leu-Arg, Trp-Ala-Gly-Gly-Asp-Ala-Ser-Gly-Glu, and
(b) herbal extracts selected from hops extract, balm mint extract, camomile extract, valerian extract, passion flower extract, lavender extract, schizandra extract, green oats extract, kava-kava extract, St. John's wort extract, griffonia extract, skullcap extract, ginger extract, orange blossom extract or mixtures thereof,
**characterized in that** said composition contains in 125 g of said composition said ingredient (a) as theanine in an amount of 0.004 % to 0.32 % by weight or as Tyr-Leu-Gly-Tyr-Leu-Glu-Gln-Leu-Leu-Arg in an amount of 0.0008 % to 0.024 % by weight or as Trp-Ala-Gly-Gly-Asp-Ala-Ser-Gly-Glu in an amount of 0.4 % to 4 % by weight, and said ingredient (b) in an amount of from 0.001 to 1 % by weight and that said composition shows an increased relaxation effect and thus supports a better sleep.

2. Composition according to claim 1, **characterized in that** said composition comprises a mixture of at least two compounds of the group of compounds listed in claim 1 for ingredient (a).

3. Composition according to claims 1-2, **characterized in that** said composition is an aqueous beverage.

4. Composition according to claim 3, **characterized in that** said composition optionally contains ingredients common in the field of nutritional beverages, such as milk, sugar or sugar substituents such as saccharin or aspartame, flavours, colorants such as β-carotin, vitamins, carbon dioxide and antioxidants and other stabilizers.

5. Composition according to claims 3 and 4, **characterized in that** said composition contains the relevant level of functional ingredients for providing measurable relaxation in a small quantity of liquid, preferably 80-150 g and more preferably 125 g, and still maintains a pleasurable taste.

6. Composition according to any of the preceding claims, **characterized in that** said composition contains (a) from 0.002 % to 0.004 % by weight of said composition of Tyr-Leu-Gly-Tyr-Leu-Glu-Gln-Leu-Leu-Arg and (b) 0.45 % by weight of said composition of balm mint extract and 0.2 % by weight of said composition of hops extract.
